# EUROPEAN PATENT APPLICATION

(11) **EP 4 269 424 A1**
(43) Date of publication of application: **01.11.2023**
(21) Application number: 22169684.2
(22) Date of filing: 25.04.2022
(51) Int. Cl.: C07K 7/06, C07K 14/08, A61P 31/12

(54) **NOVEL ANTIVIRAL COMPOUNDS AND USE THEREOF**

(71) Applicant: Københavns Universitet, 1165 København K (DK)
(72) Inventor: NILSSON, Jakob, 2200 Köpenhamn N (DK); ÖVERBY WERNSTEDT, Anna, 901 85 Umeå (SE); IVARSSON, Ylva, 751 23 Uppsala (SE)
(74) Representative: Zacco Denmark A/S

(57) **Abstract**

The present invention relates to novel compounds of the general formula CP-P1-M1-Li-M2-P2, wherein CP is a cell penetrating moiety; P1 is selected from a chemical bond and a peptide chain consisting of 1-5 amino acid residues; M1 and M2 are independently selected from peptide chains consisting of 4-7 amino acid residues comprising the motif ΦXFG, wherein Φ is a an amino acid residue with a hydrophobic side chain and X is any amino acid residue; Li is a linker separating M1 and M2; and P2 is selected from a C-terminus of M2 and a peptide chain consisting of 1-5 amino acid residues. Such compounds are useful as antiviral agents.

## Description

### Field of the invention

The present invention relates to the field of new chemical compounds, in particular peptides useful in research and in therapy.

### Background

RNA viruses such as the Ebola, dengue, and coronaviruses cause a variety of diseases and constitute a continuous threat to public health. The present inventors have previously used a viral peptide discovery approach to identify peptide based interactions for a number of coronaviruses and identified a peptide disrupting the interaction between human G3BP1/2 and the SARS CoV-2 N protein. Intracellular expression of a peptide comprising three repeats of the inhibitory peptide, each peptide-repeat containing two binding motifs explained later, dampened SARS-CoV-2 infection of VeroE6 cells (Kruse et al., 2021).

Cell-Penetrating Peptides (CPPS) are known in the art and have been suggested for use in making peptides cell-permeable (Guidotti et al., 2017). However, it is notoriously unpredictable whether any cell-penetrating peptide will actually provide such properties to a peptide and whether any function of the original peptide can be preserved and exerted if the modified peptide is transferred through the cell membrane. No cell-permeable peptide drug is used in the clinic although there are several ongoing clinical trials (Muttenthaler et al., 2021) such as for example ALRN-6924 (Saleh et al., 2021).

### Summary of the invention

The present invention builds on the surprising finding that it is possible to provide an engineered peptide that inhibits the interaction between human G3BP1/2 and viral proteins with cell-permeating properties and that such a modified peptide inhibits infection of a broad range of viruses when applied externally to cells, without significant negative impact on cell viability. This surprising finding renders it possible to administer compounds according to the invention to effectively treat viral infections.

The engineered peptides according to the invention comprise two instances of the motif ΦXFG, wherein Φ is an amino acid residue with a hydrophobic side chain and X is any amino acid residue (F and G are phenylalanine and glycine according to standard one-letter code for amino acid residues). The two ΦXFG motifs are separated by a linker separating the motifs, wherein the linker preferably is the length of a peptide backbone comprising 5-10 amino acid residues. The engineered peptides according to the invention further comprise a cell penetrating moiety providing the ability of the engineered peptides to cross cell membranes and enter cells.

In a first aspect, the present invention relates to a compound of general formula (I)

CP-P1-M1-Li-M2-P2 (I)

wherein
CP is a cell penetrating moiety;
P1 is selected from a chemical bond and a peptide chain consisting of 1-5 amino acid residues;
M1 and M2 are independently selected from peptide chains consisting of 4-7 amino acid residues comprising the motif ΦXFG, wherein Φ is a an amino
acid residue with a hydrophobic side chain and X is any amino acid residue;
Li is a linker separating M1 and M2; and
P2 is selected from a C-terminus of M2 and a peptide chain consisting of 1-5 amino acid residues.

In some embodiments, Φ in M1 and M2 is independently selected from the group consisting of L, F, I, and V.

In some embodiments, Φ is L in M1 and/or Φ is I in M2

In some embodiments, M1 is selected from the group consisting of the peptide sequences LXFGDF, IXFGDF, FXFGDF, and VXFGDF.

In some embodiments, M2 is selected from the group consisting of the peptide sequences IXFGDF, LXFGDF, FXFGDF, and VXFGDF.

In some embodiments, X is T in M1 and/or M2.

In some embodiments, Li is a peptide chain of 1-20 amino acid residues, a saturated or unsaturated aliphatic hydrocarbon, a polyeter, or a pharmaceutically acceptable organic polymer.

In some embodiments, Li is a peptide chain of 5-10 amino acid residues.

In some embodiments, CP is a cell penetrating peptide, such as YGRKKRRQRRR.

In some embodiments, P1-M1-U-M2-P2 is a peptide having an amino acid sequence selected from
LTFGDFDEHEVDALASGITFGDFDDW (SEQ ID NO: 7);
LTFGDFDEHEASGITFGDFDDW (SEQ ID NO: 8);
LTFGDFDEHEALASGITFGDFDDW (SEQ ID NO: 9);
LTFGDFTGSTGSTGSTGITFGDFDDW (SEQ ID NO: 10).

In a further aspect, the present invention relates to the compound according to the invention for use in medicine. This aspect also includes the use of the compound according to the invention for use in the manufacture of a pharmaceutical composition.

In a further aspect, the present invention relates to the compound according to the invention for use in a method for prevention or treatment of a viral infection. This aspect also includes the use of the compound according to the invention for use in the manufacture of a pharmaceutical composition for use in a method for prevention or treatment of a viral infection. This aspect also includes methods for prevention or treatment of a viral infection.

In some embodiments, the viral infection is an infection by a positive stranded RNA virus.

In some embodiments, the viral infection is an infection by a flavivirus, such as West Nile virus, Langat virus, or Tick-Borne Encephalitis virus, an alphavirus, such as Chikungunya virus, or a coronavirus, such as HCov229E, Sars-CoV-2, SARS-CoV.

In some embodiments, the peptide is administered intravenously, intradermally, intraarterially, intraperitoneally, intratracheally, intranasally, intramuscularly, intraperitoneally, subcutaneously, transmucosally, transdermally, per oral, topically, locally, and/or by inhalation.

In a further aspect, the present invention relates to pharmaceutical compositions comprising the compound according to the invention and optionally pharmaceutically acceptable excipients.

### Brief description of the drawings

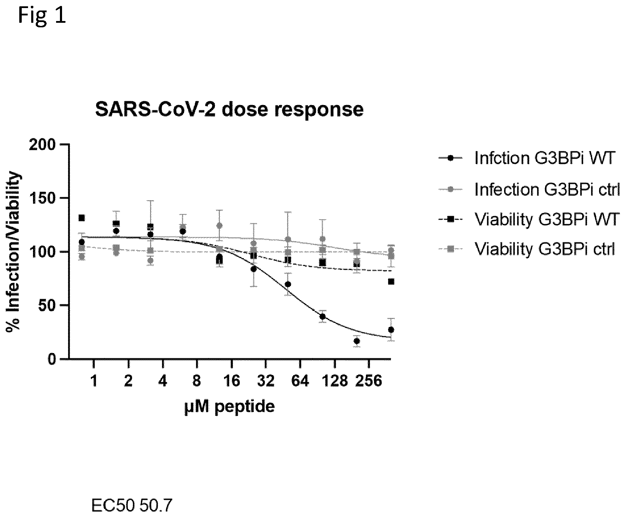

Figure 1. Cell permeable G3BP inhibitor peptides inhibit SARS-CoV-2 infection in Vero E6 cells in a dose dependent manner. Percentage SARS-CoV-2 infected cells and cell viability of cells treated with the indicated concentration of peptide 6 hours before infection compared to cells treated with an inactive version of the peptide that cannot disrupt the N-G3BP interaction. Two independent experiments (N = 7).

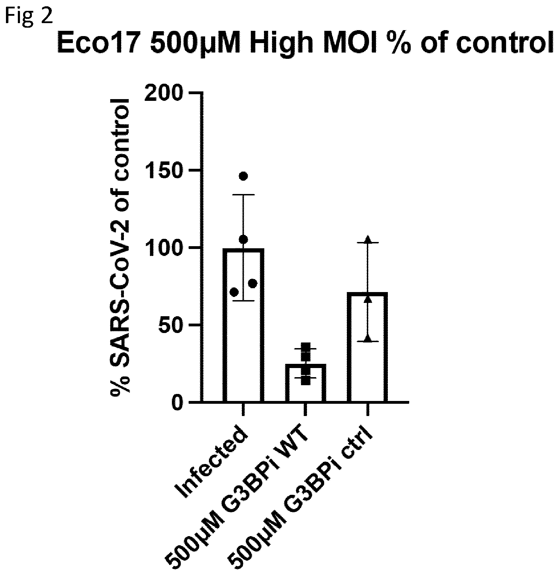

Figure 2: SARS-CoV-2 infection of fully differentiated HBEC-ALI cultures treated with G3BPi or G3BPi ctrl peptide. Apically released progeny virus were collected 72h post infection and quantified by qPCR. Infection levels are displayed as % infection of untreated control.

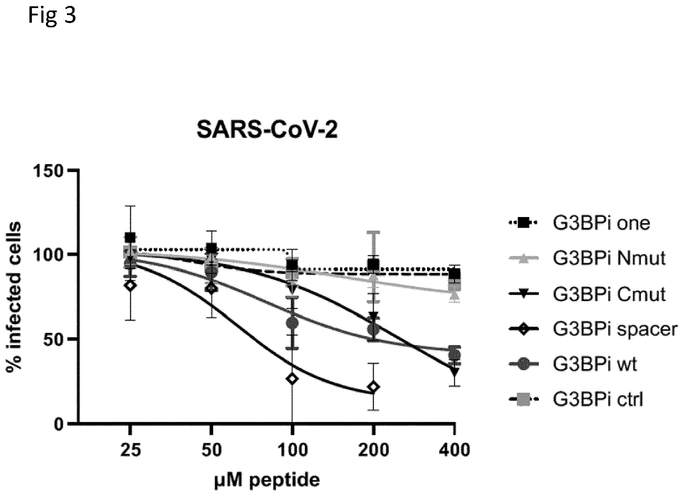

Figure 3: Inhibitory effect of modified G3BP inhibitor peptides on SARS-CoV-2 infection in a dose dependent manner in VeroE6 cells. Percentage SARS-CoV-2 infected cells treated with the indicated concentration of peptide compared to cells treated with G3BP ctrl peptide. Two independent experiments (N = 6).

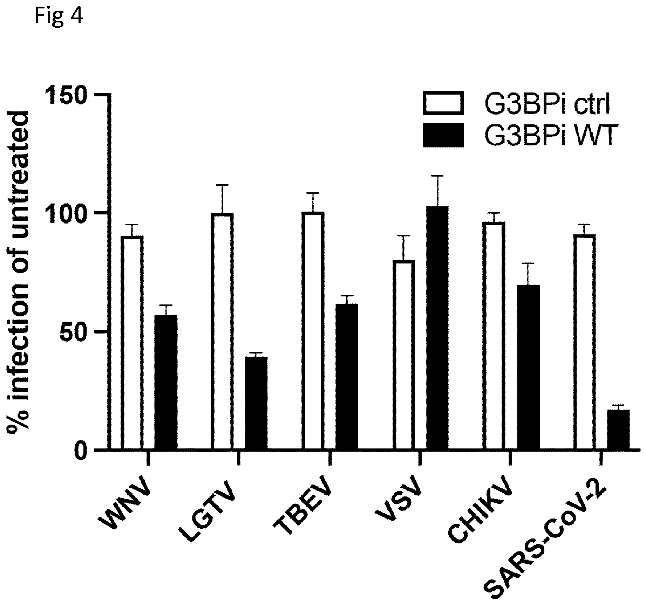

Figure 4: G3BP inhibitor peptide but not G3BP ctrl peptide inhibits a broad range of RNA viruses. Cells were treated with 200 µM peptides for 6 hours, and then infected with the indicated virus. Percentage of infected cells were compared to G3BP ctrl treated cells. Experiments were performed twice in 6 replicates (N = 12).

### Sequences

**Table 1**

| **Peptide name** | **SEQ ID NO:** | **Peptide sequence** | **Antiviral activity** |
|---|---|---|---|
| G3BPi WT | 1 | YGRKKRRQRRR**LTFG**DFDEHEVDALASG**ITFG**DFDD | Yes |
| G3BPi ctrl | 2 | YGRKKRRQRRR**LGAG**DADEHEVDALASG**IGAG**DADD | No |
| G3BPi spacer | 3 | YGRKKRRQRRR**LTFGD**FDEHEASG**ITFG**DFDD | Yes - improved |
| G3BPi one | 4 | YGRKKRRQRRR**LTFG**DFDE | No |
| G3BPi Nmut | 5 | YGRKKRRQRRR**LGAG**DADEHEVDALASG**ITFG**DFDD | No |
| G3BPi Cmut | 6 | YGRKKRRQRRR**LTFGDF**DEHEVDALASG**IGAG**DADD | Yes - reduced |
| | 7 | LTFGDFDEHEVDALASGITFGDFDDW; | |
| | 8 | LTFGDFDEHEASGITFGDFDDW | |
| | 9 | LTFGDFDEHEALASGITFGDFDDW | |
| | 10 | LTFGDFTGSTGSTGSTGITFGDFDDW | |

### Definitions and abbreviations

All terms and abbreviations used in the present disclosure are intended to have the meaning given to them by the skilled person. For the avoidance of doubt, it is noted that peptide sequences are described using the standard one-letter code, wherein

**Table 2**

| **Name** | **One Letter Code** | **Three Letter Code** |
|---|---|---|
| Alanine | A | Ala |
| Arginine | R | Arg |
| Asparagine | N | Asn |
| Aspartic Acid | D | Asp |
| Cysteine | C | Cys |
| Glutamic Acid | E | Glu |
| Glutamine | Q | Gln |
| Glycine | G | Gly |
| Histidine | H | His |
| Isoleucine | I | Ile |
| Leucine | L | Leu |
| Lysine | K | Lys |
| Methionine | M | Met |
| Phenylalanine | F | Phe |
| Proline | P | Pro |
| Serine | S | Ser |
| Threonine | T | Thr |
| Tryptophan | W | Trp |
| Tyrosine | Y | Tyr |
| Valine | V | Val |

As used in the present disclosure, X denotes any of the above amino acids.

As used in the present disclosure, Φ denotes an amino acid with a hydrophobic side chain, such as alanine (A), glycine (G), valine (V), leucine (L), isoleucine (I), phenylalanine (F), proline (P), methionine (M), and tryptophan (W).

### Detailed description

A peptide having SARS-CoV-2 N-G3BP1/2 disrupting properties comprising a double ΦXFG motif and a Cell-Penetrating Peptide (CPP) was designed (SEQ ID NO: 1). In order to evaluate the therapeutic potential of this peptide, cell penetrating peptides were designed and synthesized ("G3BPi WT", SEQ ID NO: 1) and a control peptide with the ΦXFG-motifs mutated to LGAG and IGAG ("G3BPi ctrl", SEQ ID NO: 2). Cells were pretreated with these peptides, and infection of SARS-CoV-2 was compared to that of mock treated cells.

It was found that treatment with SEQ ID NO: 1 inhibited viral infection in a dosedependent manner (Figure 1). In contrast, SEQ ID NO: 2 (G3BPi ctrl) with the ΦXFG interaction motifs mutated failed to inhibit viral infection.This indicates that the peptide according to SEQ ID NO: 1 is indeed on-target and the ΦXFG motifs crucial for the interaction with G3BP and antiviral activity. Viability of cells was not affected at the tested concentrations indicating that the peptide could be used to target viruses specifically.

To further investigate the antiviral potential of G3BPi WT against SARS-CoV-2 we used an in vitro-based 3D lung model of the human respiratory tract. Primary human bronchial epithelial cells (HBEC) were isolated from lung tissue and differentiated at an air-liquid interface (ALI) into an *in vivo*-like tissue containing basal, ciliated, and secretory cells as described in the experimental section. This state-of-the-art model closely resembles the conducting airways of the human lung and provides a unique opportunity to study coronavirus infections. Upon full differentiation, the HBEC-ALI cultures were pretreated with G3BPi WT or G3BPi ctrl followed by apical infection with SARS-CoV-2. Three days post infection, released progeny virus was determined by qPCR showing a distinct decrease in SARS-CoV-2 infection after treatment with G3BPi WT (Figure 2).

To further improve the therapeutic potential of the peptides the requirement for one or two ΦXFG motifs as well as the role of spacer sequence was explored. This confirmed that the strongest antiviral effect was observed with two ΦXFG repeats while the spacer sequence could be shortened resulting in increased antiviral activity (Figure 3).

Isothermal Titration Calorimetry (ITC) measurements on peptides lacking the Cell-Penetrating Peptide sequence (residues 1- 11 of SEQ ID NO: 1-6) showed slightly increased affinity to G3BP1 for peptides with a shorter spacer sequence between the ΦXFG -motifs, consistent with the results provided in Figure 3.

**Table 3: Affinities and thermodynamic values of G3BPi-peptides binding events inferred from ITC measurements performed at 25 °C.. ITC binding isotherms were fitted assuming a 1:2 protein (dimer concentration):peptide complex.**

| **Peptide name** | **Peptide sequence without CPP** | **Kd (µM)** |
|---|---|---|
| **G3BPi WT** | LTFGDFDEHEVDALASGITFGDFDDW | 3.6 |
| **G3BPi ctrl** | LTAADFDEHEVDALASGITAADFDDW | NB |
| **G3BPi spacer** | LTFGDFDEHEASGITFGDFDDW | 1.5 |
| **G3BPi spacer2** | LTFGDFDEHEALASGITFGDFDDW | 4.4 |
| **G3BPi spacer3** | LTFGDFTGSTGSTGSTGITFGDFDDW | 2.4 |

It can thus be concluded that disruption of interaction between G3BP1/2 and viral proteins can be provided through compounds that comprise two ΦXFGmotifs spaced some distance apart. The distance between the motifs affects the inhibitory properties to some extent, but the exact nature of the spacer appears to not be important. It is therefore contemplated that the spacer can be either a peptide sequence similar to the wild type sequence provided in e.g. SEQ ID NO: 1 or SEQ ID NO: 3, but that may also be exchanged for another peptide sequence or another molecular linker of similar length.

Based on the present inventors' knowledge of the central role of G3BP1/2 in RNA virus infection, it was further investigated if the effect on SARS CoV-2 infection could be extended to other RNA viruses. Cells were pretreated with G3BPi WT, or, G3BPi ctrl, and then infected with flaviviruses (tick-borne encephalitis virus (TBEV), Langat virus (LGTV) and West Nile virus (WNV)), alphavirus (Chikungunya virus (CHIKV)) and a rhabdovirus (vesicular stomatitis virus (VSV)) and viral infection was compared to mock treated cells. It was found that G3BPi WT peptide but not G3BPi ctrl inhibited TBEV, LGTV, WNV, and CHIKV but not VSV (Figure 4), suggesting that this interaction is important for several positive stranded RNA viruses but not the negative stranded RNA viruses.

The present invention builds on the findings above to provide compounds that disrupt interaction between viral proteins and human G3BP1 and G3BP2.

Thus, in a first aspect, the present invention relates to compound of general formula (I)

CP-P1-M1-U-M2-P2 (I)

wherein
CP is a cell penetrating moiety;
P1 is selected from a chemical bond and a peptide chain consisting of 1-5 amino acid residues;
M1 and M2 are independently selected from peptide chains consisting of 4-7 amino acid residues comprising the motif ΦXFG, wherein Φ is a an amino acid residue with a hydrophobic side chain and X is any amino acid residue;;
Li is a linker separating M1 and M2; and
P2 is selected from a C-terminus of M2 and a peptide chain consisting of 1-5 amino acid residues.

In some embodiments, Φ in M1 and M2 is independently selected from the group consisting of L, F, I, and V.

In some embodiments, Φ is L in M1 and/or Φ is I in M2

In some embodiments, X is T in M1 and/or M2.

In some embodiments, M1 is selected from the group consisting of the peptide sequences LXFGDF, IXFGDF, FXFGDF, and VXFGDF.

In some embodiments, M2 is selected from the group consisting of the peptide sequences IXFGDF, LXFGDF, FXFGDF, and VXFGDF.

In some embodiments, Li is a peptide chain of 1-20 amino acid residues, a saturated or unsaturated aliphatic hydrocarbon, or a polyeter such as polyethylene glycol (PEG) or any other pharmaceutically acceptable organic polymer. The most common non-peptide linkers are PEG chains that can have different lengths (Veronese et al., 2005). Other alternatives include poly(N-vinylpyrrolidone (PVP), polyglycerol, Poly(N-(2-hydroxypropyl) methacrylamide) (PHPMA), Polyoxazolines (POZs), PEG-based degradable polymers, e.g. poly[oligo(ethylene glycol) methyl methacrylate], polycarbonates, polysaccharides (Qi and Chilkoti, 2015).

In some embodiments, Li is a peptide chain of 1-10 amino acid residues, such as 1, 2, 3, 4, 5, 6, 7, 8, 9, or 10 amino acid residues. In some embodiments, Li is a pharmaceutically acceptable organic polymer of the same length as a peptide backbone of 1-10 amino acid residues, such as 1, 2, 3, 4, 5, 6, 7, 8, 9, or 10 amino acid residues.

In some embodiments, CP is a cell penetrating peptide. Examples of cell-penetrating peptides are HIV TAT, penetratin, polyarginine, DPV1047, MPG, Pep-1, pVEC, ARF(1-22), BPrPr(1-28), MAP, transportan, p28, VT5, BAC7, C105Y, PFVYLI, Pep-7 (Guidotti et al., 2017). A presently preferred cell-penetrating peptide is the peptide with amino acid sequence YGRKKRRQRRR (Residues 1-11 of SEQ ID NO: 1).

CP may also be a lipid moiety. Lipidation increases cell membrane permeability of peptide drugs (Menacho-Melgar et al., 2019). The lipid groups increase affinity towards the cell membrane and promotes higher cellular uptake with potential implications for intracellular delivery (Wang et al., 2002).

In some embodiments, P1-M1-U-M2-P2 is a peptide having an amino acid sequence selected from
LTFGDFDEHEVDALASGITFGDFDDW (SEQ ID NO: 7);
LTFGDFDEHEASGITFGDFDDW (SEQ ID NO: 8);
LTFGDFDEHEALASGITFGDFDDW (SEQ ID NO: 9);
LTFGDFTGSTGSTGSTGITFGDFDDW (SEQ ID NO: 10).

Peptide compounds according to the invention may be synthetically produced following established protocols (Stawikowski et al., 2012) or recombinantly produced (Kelman, 2021).

Compounds according to the invention may furthermore be stabilized by incorporation of unnatural amino acids, including L-amino acids, as shown previously (Haugaard-Kedström et al., 2021).

In a further aspect, the present invention relates to the compound according to the above for use in medicine.

In a further aspect, the present invention relates to the compound according to the above for use in a method for prevention or treatment of a viral infection.

In some embodiments, the viral infection is an infection by a positive stranded RNA virus.

In some embodiments, the viral infection is an infection by a flavivirus, such as West Nile virus, Langat virus, or Tick-Borne Encephalitis virus.

In some embodiments, the viral infection is an infection by an alphavirus, such as Chikungunya virus.

In some embodiments, the viral infection is an infection by a coronavirus, such as Sars-CoV-2, SARS-CoV, or HCov229E.

In some embodiments, the coronavirus infection is an infection by Sars-CoV-2.

In some embodiments, the peptide is administered intravenously, intradermally, intraarterially, intraperitoneally, intratracheally, intranasally, intramuscularly, intraperitoneally, subcutaneously, transmucosally, transdermally, per oral, topically, locally, and/or by inhalation.

### Methods of treatment

In a further aspect, the present invention relates to methods of treating or preventing viral infection comprising administering a compound according to the invention to a subject in need thereof.

In some embodiments, the viral infection is an infection by a positive stranded RNA virus.

In some embodiments, the viral infection is an infection by a flavivirus, such as West Nile virus, Langat virus, or Tick-Borne Encephalitis virus.

In some embodiments, the viral infection is an infection by an alphavirus, such as Chikungunya virus.

In some embodiments, the viral infection is an infection by a coronavirus, such as Sars-CoV-2, SARS-CoV, HCov229E.

In some embodiments, the coronavirus infection is an infection by Sars-CoV-2.

In some embodiments, the peptide is administered intravenously, intradermally, intraarterially, intraperitoneally, intratracheally, intranasally, intramuscularly, intraperitoneally, subcutaneously, transmucosally, transdermally, per oral, topically, locally, and/or by inhalation.

### Pharmaceutical compositions

In a further aspect, the present invention relates to a pharmaceutical composition comprising the compound according to the invention, and optionally pharmaceutically acceptable excipients and carriers.

In some embodiments, the pharmaceutical composition is adapted to be administered intravenously, intradermally, intraarterially, intraperitoneally, intratracheally, intranasally, intramuscularly, intraperitoneally, subcutaneously, transmucosally, transdermally, per oral, topically, locally, and/or by inhalation.

The pharmaceutically acceptable compositions according to the invention may comprise a therapeutically-effective amount of at least one compound according to the invention as an active ingredient, optionally combined with one or more additional therapeutically active ingredients, such as additional antiviral agents, formulated together with one or more pharmaceutically acceptable excipients. The active ingredients and excipient(s) may be formulated into compositions and dosage forms according to methods known in the art. The pharmaceutical compositions of the present invention may adapted to be administered intravenously, intradermally, intraarterially, intraperitoneally, intratracheally, intranasally, intramuscularly, intraperitoneally, subcutaneously, transmucosally, transdermally, per oral, topically, locally, and/or by inhalation.

The medicaments, pharmaceutical compositions or therapeutic combinations according to the present invention may be in any form suitable for the application to humans and/or animals, preferably humans including infants, children and adults and can be produced by standard procedures known to those skilled in the art. The medicament, (pharmaceutical) composition or therapeutic combination can be produced by standard procedures known to those skilled in the art, e.g. from "Pharmaceutics: The Science of Dosage Forms", Second Edition, Aulton, M.E. (ED. Churchill Livingstone, Edinburgh (2002); "Encyclopedia of Pharmaceutical Technology", Second Edition, Swarbrick, J. and Boylan J.C. (Eds.), Marcel Dekker, Inc. New York (2002); "Modern Pharmaceutics", Fourth Edition, Banker G.S. and Rhodes C.T. (Eds.) Marcel Dekker, Inc. New York 2002 y "The Theory and Practice of Industrial Pharmacy", Lachman L., Lieberman H. And Kanig J. (Eds.), Lea & Febiger, Philadelphia (1986). The respective descriptions are hereby incorporated by reference and form part of the disclosure.

An effective dose of the compound according to the invention may include a "therapeutically effective dose or amount" or a "prophylactically effective dose or amount" as defined above. A therapeutically effective amount may vary according to factors such as the disease state, age, sex, and weight of the individual, and the ability to elicit a desired response in the individual. A therapeutically effective dose/amount is also one in which any toxic or detrimental effects are outweighed by the therapeutically beneficial effects. A "prophylactically effective dose/amount" refers to an amount effective, at dosages and for periods of time necessary, to achieve the desired prophylactic result. Typically, since a prophylactic dose is used in subjects prior to or at an earlier stage of disease, the prophylactically effective amount will be less than the therapeutically effective amount.

### Use of compounds of the invention in the manufacture of pharmaceutical compositions

In a further aspect, the present invention relates to the use of a compound according to the invention in the manufacture of a pharmaceutical composition according to the invention. Such pharmaceutical compositions are useful in methods for prevention or treatment of viral infections, as disclosed herein.

All references cited herein are incorporated by reference in their entirety. The examples and experimental protocols disclosed herein are for the sole purpose of illustrating the invention and how to enable the skilled person to work the invention. They shall not be construed as limiting the scope of the invention, which is that of the appended claims.

### Experimental

### Cells and viruses

VeroB4 cells were cultured in medium 199/EBSS (HyClone) supplemented with 10 % FBS (HyClone), 100 units/mL penicillin, and 100 µg/mL streptomycin (Gibco). VeroE6 cells were cultured in DMEM (Sigma) supplemented with 5 % FBS (HyClone), 100 units/mL penicillin, and 100 µg/mL streptomycin (Gibco). SARS-CoV-2 (SARS-CoV-2/01/human2020/SWE accession no/GeneBank no MT093571.1, provided by the Public Health Agency of Sweden) was used at passage number 4 and was cultured and titrated in VeroE6 cells. TBEV (Torö-2003, GenBank Accession no. DQ401140.3, an infectious clone was used at passage number 2), WNV (isolated in 2003 in Israel WNV_0304h_ISR00, passage number 5, provided by the Public Health Agency of Sweden), LGTV (TP21, provided by Gerhard Dobler), VSV and CHIKV (CHIKV LR2006OPY1, kind gift of Andres Merits) virus was grown and cultured in VeroB4 cells.

### Viral infections and staining

Cells were treated with the indicated peptides, or mock treated for 6 h before infection with the indicated virus for 1 h at 37°C at 5 % CO₂ (SARS-CoV-2, VSV and CHIKV MOI:0.05, TBEV, WNV and LGTV MOI:0.1 for 24 h), then inoculum was replaced with DMEM supplemented with 2 % FBS, 100 units/mL penicillin, and 100 µg/mL. Cells were incubated for 16 h (SARS-CoV-2, CHIKV and VSV) or 24 h (TBEV, LGTV and WNV) at 37°C at 5 % CO₂ before fixation using 4 % formaldehyde. Cells were then permeabilized using 0.5 % Triton X-100, 20 mM glycine in PBS. Virus infected cells were stained using monoclonal antibodies (rabbit monoclonal to SARS-CoV-2 Nucleocapsid: 1:500 Sino Biological Inc., 40143-R001, TBEV and LGTV: mouse monoclonal 1786 1:1000 PMID: 7817895, WNV: mouse monoclonal to Anti-Flavivirus Group Antigen Antibody, clone D1-4G2-4-15 1:1000, CHIKV: mouse monoclonal to dsRNAJ2 Scicons 10010500), and secondary anti-mouse or anti-rabbit Alexa555 conjugated antibodies (1:1000 Thermo Fisher Scientific), nuclei were counterstained by DAPI. Number of nuclei and infected cells were quantified using TROPHOS Plate RUNNER HD^{®}. Percentage infection to control was calculated by normalizing to the infection of mock treated cells.

### Primary HBEC air-liquid interface (ALI) cultures

Primary human bronchial epithelial cells (HBEC) were isolated from lung tissue from a patient who underwent thoracic surgery at the University Hospital, Umeå, Sweden, with ethical permission by the local Ethics Review Board (Dnr. 2021-00317). HBEC were grown in Bronchial Epithelial Cell Medium (BEpiCM, SC3211-b, ScienCell) with recommended supplements (SC3262, ScienCell) and 100 units/mL of penicillin and 10 µg/mL of streptomycin (PeSt). For differentiation 75.000 cells were seeded onto a 6 mm semipermeable transwell insert (0.4 µm Pore Polyester Membrane Insert, Corning) in differentiation media (DMEM:BEpiCM 1:1 supplemented with 52 µg/ml bovine pituitary extract, 0.5 µg/ml hydrocortisone, 0.5 ng/ml human recombinant epidermal growth factor, 0.5 µg/ml epinephrine, 10 µg/ml transferrin, 5 µg/ml insulin, 50 nM retinoic acid (all from Sigma Aldrich), and PeSt as described (Danahay et al., 2002). Media was replaced three times per week, 250 µl to apical chambers and 700 µl to basal chambers, with addition of fresh retinoic acid to the media shortly before usage. At day 7, media was removed from the apical side and cells were grown at airliquid interface for an additional two weeks to reach full differentiation. Differentiation of the HBEC were assessed using light microscopy focusing on epithelial morphology, presence of ciliated cells, and mucus production. Presence of ciliated cells and goblet cells was also determined with IF using antibodies directed against acetylated-tubulin and muc5AC.

### Virus infection of HBEC ALI-cultures

The patient isolate SARS-CoV-2/01/human/2020/SWE (MT093571.1) was kindly provided by the Public Health Agency of Sweden. The viruses were propagated in Vero-E6 cells once and titrated by plaque assay. 4h prior to infection, the apical sides of the fully differentiated HBEC ALI-cultures (day 24 of differentiation) were rinsed three times with PBS and 100µl of 500µM SFG3BP or 500µM SFG3BP_cont (diluted in PBS) was added and the HBEC ALI-cultures were incubated at 37 °C and 5 % CO2. Immediately prior to infection, the cells were washed once with PBS and 4.5×104 PFU of SARS-CoV-2, corresponding to an approximate MOI of 0.2 was added to the apical compartment in a total volume of 100 µl infection medium (DMEM + PeSt). Cells were incubated at 37 °C in 5 % CO2 for 2.5 h before the inoculum was removed and the cells washed with PBS to remove residual medium. The HBEC-ALI cultures were treated with fresh peptides 24h and 48h post infection with the indicated concentrations for 1h at 37 °C in 5 % CO2. 72h post infection produced and secreted virus were collected from the apical compartment by addition of 100 µl warm PBS followed by an incubation for 1 h at 37 °C and 5 % CO2.

### Quantification of progeny virus

The produced and secreted virus was quantified by qPCR. RNA was extracted from 50 µl apical sample using QIAamp Viral RNA kit (Qiagen) according to the manufacturer's instructions and cDNA was synthesized using High-Capacity cDNA Reverse Transcription Kit (Applied Biosystems). Quantification was done on a StepOnePlusTM Real-Time PCR System (Applied Biosystems) using the qPCRBIO Probe Mix Hi-ROX (PCR biosystems) and SARS-CoV-2 primers (Forward: GTC ATG TGT GGC GGT TCA CT, Reverse: CAA CAC TAT TAG CAT AAG CAG TTG T) and probe (FAM - CAG GTG GAA CCT CAT CAG GAG ATG C - BHQ) specific for viral RdRp (Corman et al., 2020). Forward and reverse primer are modifications of Corman et al. made by Magnus Lindh at Sahlgrenska University Hospital, Gothenburg.

***Isothermal Titration Calorimetry (ITC).*** Peptides were purchased from Peptide 2.0 Inc (Chantilly. VA, USA). The purity obtained in the synthesis was 95 - 98 % as determined by high performance liquid chromatography (HPLC) and subsequent analysis by mass spectrometry. Prior to ITC experiments both the protein and the peptides were extensively dialyzed against 50 mM sodium phosphate, 150 mM NaCl, 0.5 mM TCEP, pH 7.5. All ITC experiments were performed on an Auto-iTC200 instrument (Microcal, Malvern Instruments Ltd.) at 25 °C. Both peptide and G3BP1 concentrations were determined using a spectrometer by measuring the absorbance at 280 nm and applying values for the extinction coefficients computed from the corresponding sequences by the ProtParam program (http://web.expasy.org/protparam/). The peptides at approximately 100/200 µM concentration were loaded into the syringe and titrated into the calorimetric cell containing the G3BP1 at ~ 10/20 µM. The reference cell was filled with distilled water. In all assays, the titration sequence consisted of a single 0.4 µl injection followed by 19 injections, 2 µl each, with 150 s spacing between injections to ensure that the thermal power returns to the baseline before the next injection. The stirring speed was 750 rpm. Control experiments with the peptides injected in the sample cell filled with buffer were carried out under the same experimental conditions. These control experiments showed heats of dilution negligible in all cases. The heats per injection normalized per mole of injectant *versus* the molar ratio [peptide]/[G3BP1] were fitted to a single-site model. Data were analysed with MicroCal PEAQ-ITC (version 1.1.0.1262) analysis software (Malvern Instruments Ltd.).

### References

Corman et al. (2020). Detection of 2019 novel coronavirus (2019-nCoV) by real-time RT-PCR. Euro Surveill, 25(3)*.*
Danahay et al. (2002, Feb). Interleukin-13 induces a hypersecretory ion transport phenotype in human bronchial epithelial cells. Am J Physiol Lung Cell Mol Physiol, 282(2), L226-L236.
Guidotti et al. (2017). Cell-Penetrating Peptides: From Basic Research to Clinics. Trends in Pharmacological Sciences, 38(4), 406-424.
Haugaard-Kedström et al. (2021, Feb 11). A High-Affinity Peptide Ligand Targeting Syntenin Inhibits Glioblastoma. J Med Chem, 64(3), 1423-1434.
Kelman, O. a. (Ed.). (2021). Methods in Enzymology (Vol. 660; Recombinant Protein Expression: Eukaryotic Hosts). Cambridge, MA, United States: Academic Press (Elsevier).
Kruse et al. (2021, Nov 19). Large scale discovery of coronavirus-host factor protein interaction motifs reveals SARS-CoV-2 specific mechanisms and vulnerabilities. Nat Commun, 12(1), 6761.
Menacho-Melgar et al. (2019, Feb 10). A review of lipidation in the development of advanced protein and peptide therapeutics. J Control Release., 295, 1-12.
Muttenthaler et al. (2021). Trends in peptide drug discovery. Nat Rev Drug Discov, 20(4), 309-325.
Qi and Chilkoti. (2015, Oct). Protein-polymer conjugation-moving beyond PEGylation. Curr Opin Chem Biol, 28, 181-193.
Saleh et al. (2021, Oct 1). Phase 1 Trial of ALRN-6924, a Dual Inhibitor of MDMX and MDM2, in Patients with Solid Tumors and Lymphomas Bearing Wildtype TP53. Clin Cancer Res, 27(19), 5236-5247.
Stawikowski et al. (2012, Aug 1). Introduction to Peptide Synthesis. Current Protocols in Protein Science, 69, 18.1.1-18.1.13. doi: 10.1002/0471140864. ps1801s69
Veronese et al. (2005, Nov 1). PEGylation, successful approach to drug delivery. Drug Discov Today, 10(21), 1451-1458.
Wang et al. (2002, May). Structure-activity relationship of reversibly lipidized peptides: studies of fatty acid-desmopressin conjugates. Pharm Res, 19(5), 609-614.

## Claims

1. A compound of general formula (I)
CP-P1-M1-U-M2-P2 (I)
wherein
CP is a cell penetrating moiety;
P1 is selected from a chemical bond and a peptide chain consisting of 1-5 amino acid residues;
M1 and M2 are independently selected from peptide chains consisting of 4-7 amino acid residues comprising the motif ΦXFG, wherein Φ is a an amino acid residue with a hydrophobic side chain and X is any amino acid residue;
Li is a linker separating M1 and M2; and
P2 is selected from a C-terminus of M2 and a peptide chain consisting of 1-5 amino acid residues.

2. The compound according to claim 1, wherein Φ in M1 and M2 is independently selected from the group consisting of L, F, I, and V.

3. The compound according to any one of the preceding claims, wherein Φ is L in M1 and/or Φ is I in M2

4. The compound according to any one of the preceding claims, wherein M1 is selected from the group consisting of the peptide sequences LXFGDF, IXFGDF, FXFGDF, and VXFGDF.

5. The compound according to any one of the preceding claims, wherein M2 is selected from the group consisting of the peptide sequences IXFGDF, LXFGDF, FXFGDF, and VXFGDF.

6. The compound according to any one of the preceding claims, wherein X is T in M1 and/or M2.

7. The compound according to any one of the preceding claims, wherein Li is a peptide chain of 1-20 amino acid residues, a saturated or unsaturated aliphatic hydrocarbon, a polyeter, or a pharmaceutically acceptable organic polymer.

8. The compound according to any one of the preceding claims, wherein Li is a peptide chain of 5-10 amino acid residues.

9. The compound according to any one of the preceding claims, wherein CP is a cell penetrating peptide, such as YGRKKRRQRRR.

10. The compound according to any one of the preceding claims, wherein P1-M1-Li-M2-P2 is a peptide having an amino acid sequence selected from
LTFGDFDEHEVDALASGITFGDFDDW (SEQ ID NO: 7);
LTFGDFDEHEASGITFGDFDDW (SEQ ID NO: 8);
LTFGDFDEHEALASGITFGDFDDW (SEQ ID NO: 9);
LTFGDFTGSTGSTGSTGITFGDFDDW (SEQ ID NO: 10).

11. The compound according to any one of the preceding claims for use in medicine.

12. The compound for use according to claim 9, for use in a method for prevention or treatment of a viral infection.

13. The compound for use according to claim 13, wherein the viral infection is an infection by a positive stranded RNA virus.

14. The compound for use according to claim 13, wherein the viral infection is an infection by a flavivirus, such as West Nile virus, Langat virus, or Tick-Borne Encephalitis virus, an alphavirus, such as Chikungunya virus, or a coronavirus, such as HCov229E, Sars-CoV-2, SARS-CoV.

15. The compound for use according to any one of claims 11-14, wherein the peptide is administered intravenously, intradermally, intraarterially, intraperitoneally, intratracheally, intranasally, intramuscularly, intraperitoneally, subcutaneously, transmucosally, transdermally, per oral, topically, locally, and/or by inhalation.
